# EUROPEAN PATENT APPLICATION

(11) **EP 3 438 281 A1**
(43) Date of publication of application: **06.02.2019**
(21) Application number: 17775109.6
(22) Date of filing: 28.03.2017
(51) Int. Cl.: C12Q 1/68, C12N 15/09

(54) **STRESS BIOMARKER**

(30) Priority: 28.03.2016 JP 2016064686
(71) Applicant: Osaka University, Suita-shi, Osaka 565-0871 (JP)
(72) Inventor: NISHIDA, Kohji, Suita-shi Osaka 565-0871 (JP); KUMANOGOH, Atsushi, Suita-shi Osaka 565-0871 (JP); HASHIDA, Noriyasu, Suita-shi Osaka 565-0871 (JP)
(74) Representative: Hoffmann Eitle
(86) International application number: PCT/JP2017/012690
(87) International publication number: WO 2017/170572

(57) **Abstract**

The principal purpose of the present invention is to provide a novel stress biomarker that makes it possible to conveniently and accurately assess a state of stress. In addition, the present invention provides a diagnosis kit containing a reagent capable of detecting said biomarker, and a diagnosis method that uses said biomarker. It is possible to use mitochondrial DNA included in a biological fluid as the stress biomarker.

SEQUENCE LISTING

<110>  Osaka University

<120>  Stress biomarker

<130>  17031WO

<150>  JP2016-064686
<151>  2016-03-28

<160>  6

<170>  PatentIn version 3.5

<210>  1
<211>  20
<212>  DNA
<213>  Artificial Sequence

<220>
<223>  forward primer for cytochrome b

<400>  1
atgaccccaa tacgcaaaat                                                   20


<210>  2
<211>  20
<212>  DNA
<213>  Artificial Sequence

<220>
<223>  reverse primer for cytochrome b

<400>  2
cgaagtttca tcatgcggag                                                   20


<210>  3
<211>  20
<212>  DNA
<213>  Artificial Sequence

<220>
<223>  forward primer for COXIII

<400>  3
atgacccacc aatcacatgc                                                   20


<210>  4
<211>  20
<212>  DNA
<213>  Artificial Sequence

<220>
<223>  reverse primer for COXIII

<400>  4
atcacatggc taggccggag                                                   20

<210>  5
<211>  20
<212>  DNA
<213>  Artificial Sequence

<220>
<223>  forward primer for NADH

<400>  5
atacccatgg ccaacctcct                                                   20


<210>  6
<211>  20
<212>  DNA
<213>  Artificial Sequence

<220>
<223>  reverse primer for NADH

<400>  6
gggcctttgc gtagttgtat                                                   20

## Description

### TECHNICAL FIELD

The present invention relates to a stress biomarker that makes it possible to accurately evaluate a stress condition.

### BACKGROUND ART

It is known that physical or psychological stress associated with a lifestyle habit, a social structure, a living environment, aging or the like may cause various diseases. Due to various physical, chemical, physiological, biological, psychological or social stress stimuli, endogenous stress substances are generated in the human body, and the immunosensor responds to the endogenous stress substances, thus causing persistent inflammation. As a result, various diseases such as infectious diseases, autoimmune diseases, lifestyle related diseases, cardiovascular diseases, neurodegenerative diseases, motor and sensory organ diseases may be developed. Since stress may cause various diseases as described above, accurate evaluation of a stress condition is extremely important in health care and prevention or treatment of disease.

As conventional stress evaluation methods, evaluation methods based on written surveys such as questionnaires have been mainly carried out. However, such methods are subjective stress evaluation methods, and lack objectivity. In addition, as other stress evaluation methods, some of applications of brain waves (α waves) and acceleration pulse waveforms have been put to practical use. However, data obtained by such an evaluation method does not give a quantitative indication of the amount of stress. In addition, it has been reported that not only disease but also various stresses in daily life increase the level of stress substances such as cortisol and catecholamine in blood, and the level of these substances may reflect a stress condition, but stress evaluation using these substances is still at a research stage, has a long way to go before being put to practical use, and has the problem of low detection sensitivity.

Recently, as a method for evaluating a stress condition, a method using a biomarker has been proposed.

For example, Patent Document 1 discloses a method in which using messenger RNA derived from peripheral blood of a subject, a chronic stress condition of the subject is evaluated based on a result of analyzing expression of a marker gene.

In addition, for example, Patent Document 2 discloses a method in which using messenger RNA derived from peripheral blood of a subject, a movement-caused stress condition of the subject is evaluated based on a result of analyzing expression of a marker gene selected from Table 1.

Further, for example, Patent Document 3 discloses a biomarker for stress-related disease which is detected from a sample including biological fluid of urine, blood, saliva or cerebrospinal fluid taken from a mammal with stress-related disorder, the biomarker being at least one compound selected from a metabolic compound in which the m/z value in negative charge or positive charge electrospray ionization mass spectrometry is given by a specific value, or a precursor compound thereof.

However, in none of conventional techniques, mitochondrial DNA is applied as a stress biomarker.

### PRIOR ART DOCUMENT

### PATENT DOCUMENTS

Patent Document 1: Japanese Patent Laid-open Publication No. 2007-306883
Patent Document 2: Japanese Patent Laid-open Publication No. 2008-54590
Patent Document 3: Japanese Patent Laid-open Publication No. 2012-47735

### SUMMARY OF THE INVENTION

### PROBLEMS TO BE SOLVED BY THE INVENTION

An object of the present invention is to provide a novel stress biomarker that makes it possible to conveniently and accurately evaluate a stress condition. The present invention also provides a diagnostic kit containing a reagent capable of detecting said biomarker, and a diagnosis method that uses said biomarker.

### MEANS FOR SOLVING THE PROBLEMS

The present inventors have extensively conducted studies for achieving the above-mentioned object. As a result, the present inventors have found that when a human is under a stress condition, the amount of mitochondrial DNA (mtDNA) in living body fluid is significantly larger than that when the human is free from stress. The present inventors have found that a condition of stress such as presence or absence or the severity of stress can be evaluated with mtDNA. In addition, the present inventors have found that since a stress condition is evaluated using mtDNA, it is possible to use a larger number of types of living body fluids including lacrimal fluid, which is not possible in conventional techniques. The present invention has been completed as a result of further conducting studies based on the above-described findings.

That is, the present invention provides an invention of the aspects described below.
Item 1. A stress biomarker including mitochondrial DNA contained in living body fluid.
Item 2. The stress biomarker according to item 1, wherein the mitochondrial DNA is contained in extracellular membrane vesicles in living body fluid.
Item 3. The stress biomarker according to item 1 or 2, wherein the mitochondrial DNA is contained in exosomes in living body fluid.
Item 4. A diagnostic kit for a stress condition which includes a reagent capable of detecting mitochondrial DNA.
Item 5. The diagnostic kit according to item 4, wherein the reagent capable of detecting mitochondrial DNA is capable of detecting at least one selected from the group consisting of cytochrome b, COXI, COXII, COXIII, NADH dehydrogenase subunit 1, NADH dehydrogenase subunit 2, NADH dehydrogenase subunit 3, NADH dehydrogenase subunit 4, NADH dehydrogenase subunit 4L, NADH dehydrogenase subunit 5, NADH dehydrogenase subunit 6, ATPase sixth subunit and ATPase eighth subunit.
Item 6. A method for examining presence or absence of stress, the method including the steps of:
   (i) measuring mitochondrial DNA in living body fluid sample obtained from a test animal; and
   (ii) detecting presence or absence of stress based on a result of the step (i).
Item 7. The method according to item 6, wherein the step (ii) is carried out based on whether a result of the step (i) which is obtained for the test animal shows a larger amount of mitochondrial DNA as compared to a result of the step (i) which is obtained for a normal control.

### ADVANTAGES OF THE INVENTION

According to the stress biomarker of the present invention, a stress condition can be conveniently and accurately evaluated. In addition, according to the present invention, the number of types of living body fluids applicable as samples is increased, and there can be provided a system capable of quantitatively evaluating a stress condition more conveniently. Further, the present invention can also be applied to development of other inspection equipment. A result obtained based on the biomarker of the present invention contributes to optimization of a treatment method associated with the disease condition of each patient. In addition, according to the present invention, there can be provided a diagnostic kit for a stress condition which includes the biomarker.

### BRIEF DESCRIPTION OF THE DRAWINGS

Fig. 1 is a schematic diagram showing the secretion and metabolic pathway of exosomes.
Fig. 2 is a graph showing the results of Experimental Example 1, i.e. changes in mtDNA concentration in lacrimal fluid of a subject before the start of consultation work, during consultation work and after the end of consultation work.
Fig. 3 is a graph showing the results of Experimental Example 2, i.e. mtDNA concentrations in serum of a subject before and after day shift and before and after night shift.
Fig. 4 is a graph showing the results of Experimental Example 2, i.e. mtDNA levels in serum of a subject before and after day shift and before and after night shift.
Fig. 5 is a graph showing the results of Experimental Example 3, i.e. mtDNA levels in serum of a group of patients with central serous chorioretinopathy and a group of normal controls.
Fig. 6 is a graph showing the results of Experimental Example 4, i.e. a relationship between a clinical finding (retinal OCT (optical coherence tomography) image) and a serum mitochondrial DNA concentration (left), and mtDNA levels in serum of a group of patients with central serous chorioretinopathy and a group of normal controls (right).

### EMBODIMENTS OF THE INVENTION

### Stress Biomarker

The stress biomarker of the present invention includes mitochondrial DNA (mtDNA) contained in living body fluid. The concentration of mtDNA in living body fluid (e.g. serum, lacrimal fluid, urine, spinal fluid) is increased when stress is given, and in the present invention, a stress condition is evaluated based on the concentration of mtDNA in living body fluid.

The mtDNA is a circular double-stranded DNA present in the matrix of mitochondria which is a cell organelle. On mtDNA, cytochrome b and cytochrome c oxidase (COX) subunits I, II, III and II; subunits 1, 2, 3, 4, 4L, 5 and 6 of NADH dehydrogenase (NADH); and ATP synthetic enzyme (ATPase) sixth subunit and eighth subunit are encoded. Detection of mtDNA as a biomarker of the present invention can be performed by detecting DNAs of these genes encoded on mtDNA. One of the specific genes may be detected as a stress biomarker, or two or more thereof may be detected in combination, and used for evaluation of a stress condition. For example, COXIII, NADH dehydrogenase, cytochrome b and the like can be suitably used for detection of mtDNA which is the stress biomarker of the present invention.

In addition, since the above-mentioned series of proteins are encoded on circular mtDNA, the behavior of mtDNA can be accurately detected by using as an indicator a DNA which encodes any of the proteins.

The source of living body fluid is not particularly limited, and any animal can be used. More specific examples of the animal include humans, and mammals other than humans. Examples of the mammal other than humans include rodents such as mice, rats, hamsters and guinea pigs, and laboratory animals such as rabbits; domestic animals such as pigs, cows, goats, horses and sheep; pets such as dogs and cats; primates such as humans, monkeys, orangutans and chimpanzees. In the present invention, the source of living body fluid is preferably a primate, more preferably a human.

In the present invention, the living body fluid is not particularly limited as long as it is a liquid which is present in a living body and can be taken and which contains mtDNA. Specific examples of the living body fluid include body fluids such as lacrimal fluid, hydatoid, vitreous humor, blood samples (whole blood, serum, plasma), sweat, urine, spinal fluid, saliva and bronchoalveolar lavage, and preferred examples thereof include lacrimal fluid, hydatoid, vitreous humor, blood samples, urine and spinal fluid. Further preferred examples thereof include lacrimal fluid, blood samples and urine from the viewpoint of low invasiveness in collection.

For example, when the living body fluid is lacrimal fluid in the present invention, lacrimal fluid is obtained by taking the lacrimal fluid directly from the eye with a dropper, a glass capillary or the like. In addition, the lacrimal fluid may be one obtained by washing the eye with a solution, and recovering the solution after washing. As the solution after washing the eye, mention is made of, for example, a PBS (phosphate buffer solution) used as a wiping liquid for the lower palpebral conjunctiva, and recovered. Specifically, 30 µL of PBS is taken with a micropipette, and a subject is made to turn up. While an observation is made with a slit-lamp microscope, the PBS is dropped onto the lower palpebral conjunctiva of the subject, and mtDNA on the conjunctiva is immediately recovered as a conjunctiva wiping liquid.

In addition, for example, when the living body fluid is serum in the present invention, it is obtained by removing blood cells, and blood coagulation factors such as fibrinogen (factor I), prothrombin (factor II), factor V and factor VIII from blood (whole blood). The method for obtaining serum is not particularly limited, and serum can be obtained according to a method employed in clinical examinations and the like. For example, serum can be obtained as a supernatant obtained after blood is left standing, or a supernatant obtained by subjecting blood to centrifugal separation. In addition, prior to the detection of the stress biomarker of the present invention, a pretreatment utilizing filtration with a filter or a column may be performed for removing contaminants such as serum proteins as necessary. Examples of the serum protein include albumin, transferrin, haptoglobin, transthyretin, α1 antitrypsin, α2 macroglobulin, α1-acid glycoprotein, immunoglobulin G (IgG), immunoglobulin A (IgA), immunoglobulin M (IgM), complement C3, apolipoprotein AI and apolipoprotein AII.

In addition, when the living body fluid is urine, an error depending on a measurement time is corrected by, for example, using urine in the early morning, so that stabilization of test results and improvement of correlation with a stress condition are expected.

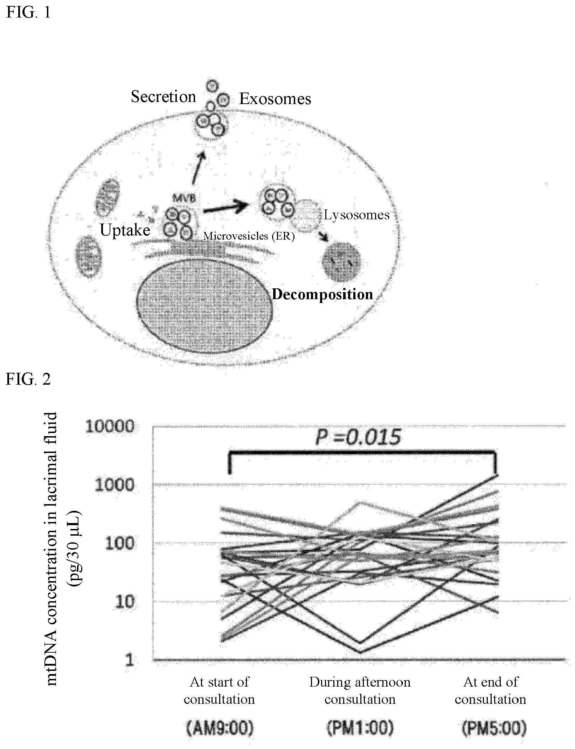

More preferably, mtDNA localized in an extracellular membrane vesicle fraction in living body fluid is used as the biomarker of the present invention. The extracellular membrane vesicle contains exosomes and microvesicles called ectosomes, and it is more preferable that mtDNA localized in an exosome fraction is used as the biomarker of the present invention. The exosome is a vesicle composed of a lipid bilayer membrane with a diameter of 50 to 200 nm, and is known to include encapsulating mRNA, microRNA, and various proteins present in the cytoplasm, and have various biological activities depending on donor cells. The exosome buds toward a secretory vesicle arising from a late endosome, forms a multivesicular body (MVB), and is transported to the cell surface. MVB is fused with a cell membrane, so that the exosome which is a content of the MVB is released outside the cell. Normally, MVB is transported not only to the cell surface, but also to a lysosome, and fused with the lysosome, so that the content thereof is decomposed (see Fig. 1 for the above).

It is considered that since mtDNA is present in such an extracellular membrane vesicle having a lipid bilayer membrane, particularly in the exosome, mtDNA is protected from being decomposed by a DNA degrading enzyme (DNase) present outside the cell, and exists stably. Thus, by using mtDNA present in the exosome as a biomarker, a stress condition can be further accurately evaluated. In addition, it is known that exosomes are also contained in a large amount in serum, urine or lacrimal fluid, and it may be possible to establish a low-invasive or noninvasive evaluation system by isolating exosomes from serum, urine or lacrimal fluid, and measuring mtDNA contained therein.

The method for separating exosomes is not particularly limited as long as a sample usable for detection of mtDNA is obtained, and one of previously known methods can be appropriately selected. Exosomes can be separated by ultracentrifugation, but more conveniently, exosomes can be separated using a commercially available kit, and specific examples of the kit include ExoQuick™ Exosome precipitation solution and Exoquick-TC (Both manufactured by System Biosciences Company).

In addition, mtDNA in lacrimal fluid is also contained in fractions other than exosomes, and may be present in a free state as exposed nucleic acids, or associated with proteins.

Detection of mtDNA is performed by preparing a polynucleotide from the living body fluid or exosome fraction, and carrying out an amplification reaction with a primer using the polynucleotide as a template, or subjecting the polynucleotide to a hybridization reaction using a probe. From the viewpoint of stability and accuracy of evaluation, the polynucleotide to be prepared is preferably DNA. Preparation of the polynucleotide from living body fluid or exosome fractions can be performed by appropriately using a known method, and examples of the method include methods of phenol extraction and ethanol precipitation, and methods using glass beads. More conveniently, the polynucleotide can be prepared using a commercially available DNA extraction reagent or a DNA extraction kit. Specific examples of the DNA extraction kit include QIAamp DNA mini Kit (Qiagen K.K.).

Detection of DNA corresponding to the aforementioned protein encoded on mtDNA can be performed by a method appropriately selected from previously known methods. For detection, a polynucleotide serving as a probe or an oligonucleotide serving as a primer is normally used for measurement of the expression level or amplification of the protein. As the primer, mention is made of an oligonucleotide having a unique sequence capable of being specifically hybridized to at least a part of a target gene to amplify the gene. In addition, as the probe, mention is made of a polynucleotide having a unique sequence that is specifically hybridized to at least a part of a target gene.

These primers or probes can be designed and synthesized in accordance with a previously known method based on sequence information of a target gene, which is obtained using a program and database such as BLAST or FASTA. The length of the base sequence of the primer is normally 16 to 32 bp, preferably 18 to 30 bp, more preferably 20 to 24 bp. Specific examples of the primer set for specifically amplifying the cytochrome b gene include primer sets which are used in examples as described later, and are expressed by SEQ ID NOS: 1 and 2. In addition, specific examples of the primer set for specifically amplifying the COXIII gene include 5'-ATGACCCACCAATCACATGC-3' (forward primer: SEQ ID NO: 3); and 5'-ATCACATGGCTAGGCCGGAG-3' (reverse primer: SEQ ID NO: 4). In addition, specific examples of the primer set for specifically amplifying the NADH gene include 5'-ATACCCATGGCCAACCTCCT-3' (forward primer: SEQ ID NO: 5); and 5'-GGGCCTTTGCGTAGTTGTAT-3' (reverse primer: SEQ ID NO: 6).

Detection of mtDNA can be performed based on an amplification product obtained by a gene amplification reaction using the primer as described above, or a hybrid product obtained by a hybridization reaction using a probe.

The method for amplification of a target gene is not particularly limited, and a previously known method can be employed. Examples thereof include amplification of DNA/RNA by a polymerase chain reaction (PCR), more specifically RT-PCR, Nested PCR, real-time PCR, competitive PCR, TaqMan PCR and Direct PCR. In addition, a modified PCR method such as a LAMP (Loop-mediated isothermal Amplification) method, an ICAN (Isothermal and Chimeric primer-initiated Amplification of Nucleic Acids) method, or a RCA (Rolling Circle Amplification) method may be used.

The method for detection of an amplification product is not particularly limited as long as it is possible to determine whether the product is a desired polynucleotide or not. For example, whether or not a polynucleotide with a predetermined size is amplified can be checked by agarose gel electrophoresis. In addition, the amplification product can be detected by labeling deoxynucleotide triphosphate (dNTP) taken in the amplification product in the process of the amplification reaction, and measuring a label substance of dNTP taken in the amplification product. Examples of the label substance include fluorescent substances such as fluorescein (FITC), sulforhodamine (SR) and tetramethylrhodamine (TRITC); luminescent substances such as luciferin; and radioactive isotopes such as 32P, 35S and 1211. Alternatively, the amplification product may be quantitatively detected by an intercalator method using SYBR Green or the like.

In addition, the length of the base sequence of the probe used for detection of mtDNA is normally 20 to 250 bp, preferably 20 to 100 bp, more preferably 20 to 50 bp. In addition, the probe that is hybridized to the polynucleotide is not required to have a perfect complementary sequence as long as it can be hybridized to at least a part of the polynucleotide, and detected.

For the hybridization reaction using the probe, conditions under which the probe is specifically hybridized to a target polynucleotide (stringent conditions), for example conditions under which DNAs having homology of 90% or more are hybridized to each other, and DNAs having lower homology are not hybridized to each other, can be appropriately set based on previously known conditions. As stringent conditions, for example, washing is performed at a temperature of 40°C to 70°C, a sodium concentration of 150 to 900 mM and a pH of 6 to 8, more specifically washing is performed at 50°C with 2 × SSC (300 mM NaCl and 30 mM citric acid) and 0.1 vol% SDS.

In addition, if necessary, the probe may be labeled using a fluorescent substance such as fluorescein (FITC) or sulforhodamine (SR), tetramethylrhodamine (TRITC); a luminescent substance such as luciferin; a radioactive isotope such as 32P, 35S or 1211; an enzyme such as alkali phosphatase or horseradish peroxidase; a label substance such as biotin. By detecting the target substance in a previously known method based on the type of each label substance after the hybridization reaction, a hybridization product can be detected.

An animal (preferably a human) under a stress condition has a significantly larger amount of mtDNA contained in living body fluid (or in exosomes), i.e. a stress biomarker of the present invention, as compared to a group of normal controls free from stress. Here, the group of normal controls refers to test animals (preferably humans) that are healthy, and free from stress.

In the present invention, the stress means that various external stimuli (stressors) cause physical and mental loads, leading to occurrence of strain distortion. Examples of the stressor that may cause stress include physical/chemical stressors, biological stressors, and psychological stressors.

The physical/chemical stressor is derived from an external environment, and examples thereof include temperature, humidity, light, noises, injuries, harmful substances and air pollution. The biological stressor is derived from an ecological environment, and examples thereof include exercises, physical fatigue, psychological fatigue, overwork, insufficient sleep, malnutrition, virus infection and bacterial infection. The psychological stressor is derived from a psychological condition or a surrounding environment in social life, and examples thereof include anxiety, tension, worry, frustration, anger, fear, disappointment and conflict.

In addition, the stress in the present invention includes stress caused by internal factors such as retrogradation, aging, bad lifestyle habits, arteriosclerosis, obesity and basal metabolism itself in addition to stress caused by the above-mentioned external stimuli (stressors). These internal factors may also cause chronic inflammation.

As described above, the stress biomarker of the present invention makes it possible to accurately evaluate a stress condition.

### Diagnostic Kit

The diagnostic kit for a stress condition according to the present invention includes a reagent capable of detecting the mtDNA. When detection of mtDNA is performed based on a gene or gene fragment encoded on mtDNA, a primer that specifically amplifies the gene or gene fragment, or a probe which is specifically hybridized to the gene or gene fragment is included as a reagent capable of detecting mtDNA. Specific examples of the reagent included in the kit according to the present invention include primers shown in SEQ ID NOS: 1 to 6. These primers and probes are as described in the section of "Stress Biomarker".

Further, the reagent capable of detecting mtDNA may contain a buffer solution, a salt, a stabilizer, a preservative and the like, and may be formulated in accordance with a previously known method. Further, in addition to the reagent, the diagnostic kit according to the present invention may contain a label substance, a label substance detecting agent, a reaction diluting solution, a standard antibody, a buffer solution, a solubilizing agent, a cleaning agent, a reaction stopping solution, a control sample and the like which may be required to perform the detection of mtDNA.

In the kit according to the present invention, for example, a probe for detecting mtDNA can be used with the probe immobilized on an insolubilized carrier. Therefore, the diagnostic kit according to the present invention may also include an insolubilized carrier. The material of the insolubilized carrier is not particularly limited as long as it does not hinder detection of mtDNA, and examples thereof include polystyrene, polyethylene, polypropylene, polyester, polyacrylonitrile, polyvinyl chloride, fluororesin, crosslinked dextran, polysaccharide, paper, silicon, glass, metal and agarose. Two or more of these materials may be used in combination. The shape of the insolubilized carrier may be any of, for example, a microplate shape, a tray shape, a spherical shape, a fiber shape, a rod shape, a disk shape, a container shape, a cell shape, a test tube shape and the like.

For example, a probe that can be specifically hybridized to a gene encoded on mtDNA can be immobilized on the insolubilized carrier to obtain a DNA chip to be used for detection of a stress condition. Immobilization of the probe on the insolubilized carrier can be performed in accordance with a previously known method. In addition, when probes for mtDNA are immobilized on a carrier at different concentrations and at equal intervals, and hybridized to mtDNA, mtDNA can be semi-quantitatively detected.

### Evaluation method based on the amount of mtDNA in living body fluid

The present invention provides a method for examining presence or absence of stress using mtDNA in living body fluid, which is the stress biomarker, the method including the steps of:
(i) measuring mitochondrial DNA in a living body fluid sample obtained from a test animal; and
(ii) detecting presence or absence of stress based on a result of the step (i).

Measurement of the living body fluid sample and mtDNA in the living body fluid sample as described in the step (i) can be performed in accordance with the procedure described in the section of "Stress Biomarker".

In addition, the detection step described in the step (ii) can be carried out based on whether a result of the step (i) which is obtained for the test animal shows a larger amount of mitochondrial DNA as compared to a result of the step (i) which is obtained for a group of normal controls. Here, the group of normal controls is as described in the section of "Stress Biomarker".

In the present invention, the phrase "the amount of mitochondrial DNA is larger than that in the group of normal controls" means that it is equal to or greater than the average value of the amount of mitochondrial DNA in living body fluid of the group of normal controls + 2 × SD (standard deviation), and the phrase "the amount of mitochondrial DNA is markedly larger than that in the group of normal controls" means that it is equal to or greater than the average value of the amount of mitochondrial DNA in living body fluid of the group of normal controls + 4 × SD. The average value and SD of the amount of mitochondrial DNA in living body fluid of the group of normal controls can be determined by measuring the amount of mitochondrial DNA in living body fluid of, for example, a group of 24 or more normal controls. In addition, for the amount of mitochondrial DNA in serum of the group of normal controls (humans), the present inventors have found that the average value + 2 × SD is 13.1 ng/mL, and the average value + 4 × SD is 16.9 ng/mL. Therefore, when the test animal is a human, and the living body fluid is serum, it can be determined that "the amount of mitochondrial DNA is larger than that in normal controls" when the amount of mitochondria in serum is 13.1 ng/mL or more, and "the amount of mitochondrial DNA is markedly larger than that in normal controls" when the amount of mitochondria in serum is 16.9 ng/mL or more. In addition, for the amount of mitochondrial DNA in lacrimal fluid of the group of normal controls (humans), the present inventors have found that the average value + 2 × SD is 648.1 pg (picogram)/30 µL, and the average value + 4 × SD is 854.5 pg/30 µL. Therefore, when the test animal is a human, and the living body fluid is lacrimal fluid, it can be determined that "the amount of mitochondrial DNA is larger than that in normal controls" when the amount of mitochondria in lacrimal fluid is 648.1 pg/30 µL or more, and "the amount of mitochondrial DNA is markedly larger than that in normal controls" when the amount of mitochondria in lacrimal fluid is 854.5 pg/30 µL or more.

In addition, examples of the test animal include humans, and mammals other than humans. Examples of the mammal other than humans include rodents such as mice, rats, hamsters and guinea pigs, and laboratory animals such as rabbits; domestic animals such as pigs, cows, goats, horses and sheep; pets such as dogs and cats; primates such as humans, monkeys, orangutans and chimpanzees. In the present invention, the test animal is preferably a primate, more preferably a human.

Further, evaluation based on the stress biomarker of the present invention can be utilized not only for examination of presence or absence of stress, but also for a method for prediction of resistance to stress, a method for prediction of susceptibility to stress-related disease, a method for diagnosis of the severity of a stress condition, a method for prediction of prognosis of a stress condition, a method for screening of a therapeutic agent, a method for differential diagnosis of stress, and the like.

When resistance to stress is evaluated based on the stress biomarker of the present invention (method for prediction of resistance to stress), the level of resistance to stress is evaluated based on the result of measuring mitochondrial DNA in a living body fluid sample obtained from a test animal as in the case of the method for examining presence or absence of stress. The level of resistance to stress is evaluated in accordance with the following criterion: the resistance to the stress is low when the amount of mitochondrial DNA is larger than that in a group of normal controls, and the resistance to the stress is very low when the amount of mitochondrial DNA is markedly larger than that in a group of normal controls.

When susceptibility to stress-related disease is predicted based on the stress biomarker of the present invention (method for prediction of susceptibility to stress-related disease), the susceptibility to stress-related disease is evaluated based on the result of measuring mitochondrial DNA in a living body fluid sample obtained from a test animal as in the case of the method for examining presence or absence of stress. The susceptibility to stress-related disease is evaluated in accordance with the following criterion: the test animal is susceptible to stress-related disease when the amount of mitochondrial DNA is larger than that in a group of normal controls, and the test animal is particularly susceptible to stress-related disease when the amount of mitochondrial DNA is markedly larger than that in a group of normal controls.

The stress-related disease is a disease developed such that endogenous stress substances are generated in the human body due to stress, and an immunosensor responds to the endogenous stress substances, thus causing persistent inflammation. Examples of the stress-related disease include neurological disorders such as anxiety disorders, digestive system diseases such as gastritis, gastric ulcer and irritable bowel syndrome, bronchial asthma, cardiovascular diseases such as angina pectoris, migraine, atopic dermatitis, alopecia areata, depression, and lifestyle diseases such as obesity and metabolic syndrome.

When the severity of a stress condition is evaluated based on the stress biomarker of the present invention (method for detection of severity of a stress condition), the severity is detected based on the result of measuring mitochondrial DNA in a body fluid sample obtained from a test animal as in the case of the method for examining presence or absence of stress. The severity is detected in accordance with the following criterion: there is the possibility that the test animal has a severe stress condition when the amount of mitochondrial DNA is larger than that in a group of normal controls, and the possibility is high that the test animal has a particularly severe stress condition when the amount of mitochondrial DNA is markedly larger than that in a group of normal controls. Alternatively, the severity is detected based on the following criteria: the stress condition is severe when the biomarker value detected from the test animal is higher than the standard value of the biomarker in the stress condition.

When the prognosis of a stress condition is evaluated based on the stress biomarker of the present invention (method for prediction of prognosis of a stress condition), the prognosis is predicted based on the result of measuring mitochondrial DNA in a living body fluid sample obtained from a test animal as in the case of the method for examining presence or absence of stress. The prognosis is predicted in accordance with the following criterion: there is the possibility that the test animal has poor prognosis of a stress condition when the amount of mitochondrial DNA is larger than that in a group of normal controls, and the possibility is high that the test animal has poor prognosis of a stress condition when the amount of mitochondrial DNA is markedly larger than that in a group of normal controls. Alternatively, the prognosis is evaluated based on the following criterion: the test animal has poor prognosis of the stress condition when the biomarker value detected from the test animal is higher than the standard value of the biomarker in the stress condition.

When screening of a therapeutic agent effective for reduction of stress is performed based on the stress biomarker of the present invention, the screening is performed based on the result of measuring mitochondrial DNA in a living body fluid sample obtained from a test animal under a stress condition, which has been given the therapeutic agent, as in the case of the method for examining presence or absence of stress. The screening is performed based on the following criterion: the therapeutic agent is effective when the amount of mitochondrial DNA is smaller than that in a test animal under a stress condition, which has not been given the therapeutic agent.

When differential diagnosis of stress-related disease is performed, i.e. differentiation of disease caused by stress, based on the stress biomarker of the present invention, differentiation from similar disease is performed based on the result of measuring mitochondrial DNA in a living body fluid sample obtained from a test animal as in the case of the method for examining presence or absence of stress. The differentiation from similar disease is performed based on the following criterion: the disease is caused by stress when the amount of mitochondrial DNA is larger than the standard value of the biomarker in disease to be compared.

In any of these methods, a correlation diagram between the amount of mtDNA in living body fluid and presence or absence of a stress condition, severity of a stress condition, susceptibility to a stress condition, prognosis of a stress condition, or the like may be prepared, followed by applying the amount of mtDNA in serum of a test animal to the correlation diagram to perform evaluation.

Based on these evaluation methods, disease can be evaluated at an individual level, so that the treatment method can be optimized according to an individual disease condition.

### EXAMPLES

Hereinafter, the present invention will be described by way of test examples, but the present invention is not limited to these test examples.

All the tests were conducted with the approval of the Ethics Committee of Osaka University Hospital.

### (Example 1)

One healthy oculist, who was involved in consultation work (work overtaxing eyes) from 9 a.m. to 5 p.m., was designated as a subject. Lacrimal fluid was taken from the subject at the start of morning consultation (9 a.m.), at the start of afternoon consultation (1 p.m.) and at the end of consultation (5 p.m.). The amount of mitochondrial DNA (mtDNA) in the collected lacrimal fluid was measured by the method shown below. In addition, on different 15 consultation days in total, lacrimal fluid was taken under the same condition as described above, and the amount of mitochondrial DNA (mtDNA) in the lacrimal fluid was measured in the same manner as described above. The obtained amount of mtDNA was statistically examined.

### Preparation of lacrimal fluid sample

As a lacrimal fluid sample, 30 µL of PBS (phosphate buffer solution) used as a wiping liquid for the lower palpebral conjunctiva, and recovered was used. Specifically, 30 µL of PBS was taken with Pipetman, and a subject was made to turn up. While an observation was made with a slit-lamp microscope, the PBS was dropped onto the lower palpebral conjunctiva of the subject, and mtDNA on the conjunctiva was immediately recovered as a conjunctiva wiping liquid. The thus-obtained liquid was used as the lacrimal fluid sample.

### Separation of DNA in Lacrimal Fluid

Using QIAamp DNA mini Kit from Qiagen K.K., lacrimal fluid DNA was prepared from the obtained lacrimal fluid (5 µl) in accordance with the attached protocol. Specifically, 5 µl of the lacrimal fluid sample was digested with 415 µl of a proteolytic enzyme (56°C, 10 minutes), DNA was precipitated with 100 vol% ethanol, and the DNA was purified using a purification column attached to the kit. The obtained lacrimal fluid DNA was resuspended in DNase-free water (20 µl).

### Real Time PCR

Using SYBR Premix Ex Taq (Perfect Real Time) (manufactured by Takara Bio Inc.), real time PCR by an intercalator method using SYBR Green I was performed by ABI PRISM 7700 (Life Technologies Japan) in accordance with the attached protocol. The PCR conditions are as follows.
Stage 1 (1 cycle): 95°C for 30 seconds;
Stage 2 (40 cycles): 95°C for 5 seconds and 60°C for 30 seconds; and
Stage 3 (1 cycle): 95°C for 15 seconds, 60°C for 1 minute and 95°C for 15 seconds

Using purified DNA derived from lacrimal fluid of healthy persons, a real time standard curve was prepared for determining the mtDNA concentration. Further, a sample which did not produce a PCR product even after completion of 40 cycles of the PCR reaction (i.e. a sample in which emission of fluorescence by SYBR Green was not observed) was considered as being "undetectable".

Primers used for detection of mtDNA are shown below.

### (Cytochrome b)

Forward primer (SEQ ID NO: 1):
   5'-ATGACCCCAATACGCAAAAT-3'
Reverse primer (SEQ ID NO: 2):
   5'-CGAAGTTTCATCATGCGGAG-3'

The total amount of mtDNA in the lacrimal fluid was determined in the following manner: fluorescein Na was dropped at a known concentration to the eye surface beforehand, the fluorescence amount of fluorescein Na in the recovered lacrimal sac washing solution was measured before and after the recovery to determine the dilution ratio, and the amount of lacrimal fluid on the whole eye surface was estimated. The obtained amount of mtDNA was statistically examined by the GEE (Generalized estimating equation) method after a relative value obtained based on a calibration curve was logarithmically processed. The results are shown in the graph in Fig. 2.

Fig. 2 is a graph showing a change in concentration of mtDNA in lacrimal fluid of the subject. The graph of Fig. 2 shows that the mtDNA concentration is significantly higher at the end of consultation than at the start of consultation (p = 0.015). This indicates that stress associated with work overtaxing eyes causes a significant increase in concentration of mtDNA in lacrimal fluid as compared to the concentration of mtDNA in lacrimal fluid at the start of consultation when the subject is free from stress, and thus mtDNA in lacrimal fluid can serve as a biomarker that makes it possible to evaluate a stress condition.

### (Experimental Example 2)

Healthy hospital staff members were designated as subjects. A change in the amount of mtDNA in serum before and after day shift and before and after night shift was evaluated for the subjects by the following method. The day shift corresponds to working from 9 a.m. to 5 p.m., and the night shift corresponds to working from 5 p.m. to 9 a.m. Ten members were subjected to evaluation before and after day shift, and nine members were subjected to evaluation before and after night shift. Peripheral blood was taken from the subjects, and serum was taken by a conventional method.

### Separation of Serum DNA

Using QIAamp DNA mini Kit from Qiagen Co., Ltd., serum DNA was prepared from the serum (100 µl) in accordance with the attached protocol. Specifically, 100 µl of the serum sample was digested with 320 µl of a proteolytic enzyme (56°C, 10 minutes), DNA was precipitated with 100 vol% ethanol, and the DNA was purified using a purification column attached to the kit. The obtained serum DNA was resuspended in DNase-free water (20 µl).

### Real Time PCR

Using SYBR Premix Ex Taq (Perfect Real Time) (manufactured by Takara Bio Inc.), real time PCR by an intercalator method using SYBR Green I was performed by ABI PRISM 7700 (Life Technologies Japan) in accordance with the attached protocol. The PCR conditions are as follows.
Stage 1 (1 cycle): 95°C for 30 seconds;
Stage 2 (40 cycles): 95°C for 5 seconds and 60°C for 30 seconds; and
Stage 3 (1 cycle): 95°C for 15 seconds, 60°C for 1 minute and 95°C for 15 seconds

Using purified DNA derived from a whole cell lysate of peripheral blood mononuclear cells (PBMC) of healthy persons, a real time standard curve was prepared for determining the mtDNA concentration. Further, a sample which did not produce a PCR product even after completion of 40 cycles of the PCR reaction (i.e. a sample in which emission of fluorescence by SYBR Green was not observed) was considered as being "undetectable".

Primers used for detection of mitochondrial DNA are shown below.

### (Cytochrome b)

Forward primer (SEQ ID NO: 1):
   5'-ATGACCCCAATACGCAAAAT-3'
Reverse primer (SEQ ID NO: 2):
   5'-CGAAGTTTCATCATGCGGAG-3'

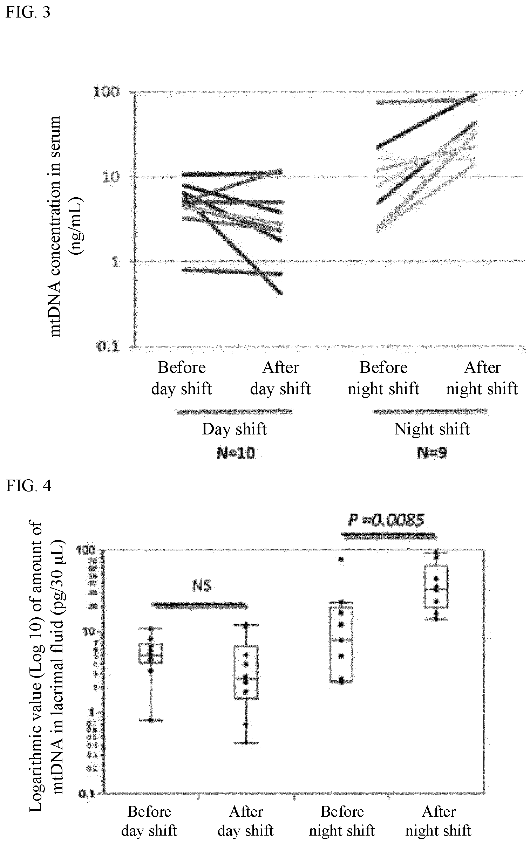

The obtained change in mtDNA concentration is shown in the graph in Fig. 3. The relative values obtained based on a calibration curve were logarithmically processed, and then statistically examined by the t-test (Paired t-test) method. The results are shown in the graph in Fig. 4.

Fig. 3 is a graph showing a change in mtDNA concentration in serum of a subject before and after day shift and before and after night shift. Fig. 4 is a graph showing an mtDNA level in serum of a subject before and after day shift and before and after night shift. The graphs in Fig. 3 and Fig. 4 show that there was no significant difference in mtDNA level in serum before and after day shift, but regarding the mtDNA level before and after night shift, the mtDNA level in serum was significantly increased after night shift (p = 0.0085). This indicates that physical or psychological stress is developed more likely in night shift than in day shift, the mtDNA level is significantly increased when the subject is given stress from night shift, and there is no significant change in mtDNA level when the subject is free from such stress. Therefore, it is apparent that mtDNA can be applied as a biomarker for stress.

### (Experimental Example 3)

It is considered that stress increases the permeability of the blood vessels of choroid, so that the barrier function is lost, and thus fluid is accumulated under the retina, resulting in development of central serous chorioretinopathy. The reason why the disease is caused by stress is that it has been reported that the same situation as described above is produced when a monkey is given a catecholamines such as adrenaline, of which concentration in blood is increased by stress, and epidemiologically people with an A-type disposition are dominant. A biomarker for detecting central serous chorioretinopathy has not been present heretofore.

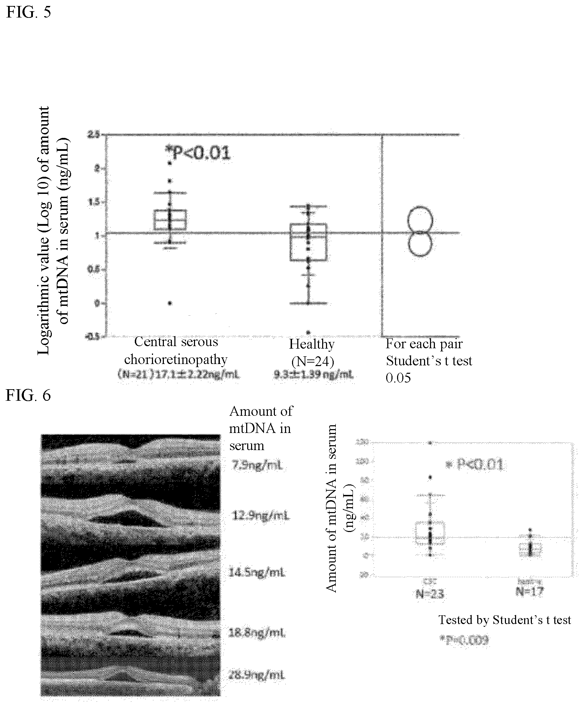

In this test, serum was taken from each of patients (21 patients) with central serous chorioretinopathy, and the amount of mtDNA in the serum was measured in the same manner as in Experimental Example 2. Serum was taken from each of healthy persons (24 persons) as a group of normal controls, and the amount of mtDNA in the serum was measured under the same conditions. The obtained value was statistically examined by the Student's t test method. The results are shown in the graph in Fig. 5.

Fig. 5 is a graph showing mtDNA concentrations in serum of a group of patients with central serous chorioretinopathy and a group of normal controls. The graph in Fig. 5 shows that the mtDNA concentration in serum in the group of patients with central serous chorioretinopathy was significantly higher than that in the group of normal controls (P < 0.01). It is apparent that since the mtDNA concentration in serum in the group of patients with stress-related disease is higher than that in the group of normal controls, mtDNA can be used as a stress biomarker for stress-related disease.

### (Experimental Example 4)

In this experimental example, the number n in the test in Experimental Example 3 was increased, and the same analysis was performed. Specifically, serum was taken from each of patients (23 patients) with central serous chorioretinopathy, and the amount of mtDNA in the serum was measured in the same manner as in Experimental Example 3. Serum was taken from each of healthy persons (24 persons) as a group of normal controls, and the amount of mtDNA in the serum was measured under the same conditions. The obtained value was statistically examined by the Student's t test method.

The results are shown in Fig. 6. The left diagram in Fig. 6 shows clinical findings (OCT (optical coherence tomography) images of the retina) and the results of the mitochondrial DNA concentration in serum for six patients with central serous chorioretinopathy. The right diagram in Fig. 6 shows the results of measuring the amount of mtDNA in serum in the patient group (CSC) and the group of normal controls (Healthy). The results show that the mtDNA concentration in serum in the group of patients with central serous chorioretinopathy considered to be caused by stress was higher than that in the group of normal controls. As is evident from the left diagram in Fig. 6, the amount of mtDNA in serum tended to increase as serous retinal detachment progressed (the amount of subretinal fluid increased). These results show that mtDNA in body fluid can be used as a stress biomarker.

SEQ ID NO: 1 is a forward primer for cytochrome b.
SEQ ID NO: 2 is a reverse primer for cytochrome b.
SEQ ID NO: 3 is a forward primer for COXIII.
SEQ ID NO: 4 is a reverse primer for COXIII.
SEQ ID NO: 5 is a forward primer for NADH dehydrogenase.
SEQ ID NO: 6 is a reverse primer for NADH dehydrogenase.

## Claims

1. A stress biomarker comprising mitochondrial DNA contained in living body fluid.

2. The stress biomarker according to claim 1, wherein the mitochondrial DNA is contained in extracellular membrane vesicles in living body fluid.

3. The stress biomarker according to claim 1 or 2, wherein the mitochondrial DNA is contained in exosomes in living body fluid.

4. A diagnostic kit for a stress condition which comprises a reagent capable of detecting mitochondrial DNA.

5. The diagnostic kit according to claim 4, wherein the reagent capable of detecting mitochondrial DNA is capable of detecting at least one selected from the group consisting of cytochrome b, COXI, COXII, COXIII, NADH dehydrogenase subunit 1, NADH dehydrogenase subunit 2, NADH dehydrogenase subunit 3, NADH dehydrogenase subunit 4, NADH dehydrogenase subunit 4L, NADH dehydrogenase subunit 5, NADH dehydrogenase subunit 6, ATPase sixth subunit and ATPase eighth subunit.

6. A method for examining presence or absence of stress, the method comprising the steps of:
(i) measuring mitochondrial DNA in living body fluid sample obtained from a test animal; and
(ii) detecting presence or absence of stress based on a result of the step (i).

7. The method according to claim 6, wherein the step (ii) is carried out based on whether a result of the step (i) which is obtained for the test animal shows a larger amount of mitochondrial DNA as compared to a result of the step (i) which is obtained for a normal control.
